# EUROPEAN PATENT APPLICATION

(11) **EP 4 230 728 A1**
(43) Date of publication of application: **23.08.2023**
(21) Application number: 21869620.1
(22) Date of filing: 08.09.2021
(51) Int. Cl.: C12N 5/0783, A61K 35/17, A61K 48/00, A61P 37/00, A61P 17/00, A61K 8/98

(54) **IMMUNE CELL PROLIFERATION/ACTIVATION KIT AND IMMUNE CELL CULTURE METHOD USING SAME PROLIFERATION/ACTIVATION KIT**

(30) Priority: 15.09.2020 KR 20200118665; 29.07.2021 KR 20210099651
(71) Applicant: Shin, Ji-Seop, Paju-si, Gyeonggi-do 10908 (KR); Shin, Woo Seop, Gyeonggi-do 13527 (KR)
(72) Inventor: Shin, Ji-Seop, Paju-si, Gyeonggi-do 10908 (KR); Shin, Woo Seop, Gyeonggi-do 13527 (KR)
(74) Representative: Beck & Rössig European Patent Attorneys
(86) International application number: PCT/KR2021/012211
(87) International publication number: WO 2022/059998

(57) **Abstract**

The present disclosure relates to an immune cell culture method and, more particularly, to an immune cell proliferation/activation kit and an immune cell culture method using the proliferation/activation kit, in which the kit not only suppresses regulatory T cells (Treg) during immune cell culture to allow any one of natural killer cells (NK cells) and T cells from among immune cells to efficiently proliferate but also efficiently amplifies and activates immune cells for a long period of time.

## Description

### Technical Field

The present disclosure relates to an immune cell culture method and, more particularly, to an immune cell proliferation/activation kit and an immune cell culture method using the proliferation/activation kit, in which the kit not only suppresses regulatory T cells (Treg) during immune cell culture to allow any one of natural killer cells (NK cells) and T cells from among immune cells to efficiently proliferate but also efficiently amplifies and activates immune cells for a long period of time.

### Background Art

NK cells are known to play an important role in the early biological defense mechanism and tumor immunity of the human body. In other words, NK cells can kill specific autologous cells, allogeneic cells, and even heterogeneous cancer cells without the process of acquiring immunity according to the expression of the major histocompatibility complex (MHC), and NK cells are particularly good at killing target cells that express fewer or no Class 1 MHCs. Therefore, NK cells can effectively kill most cancer cells that do not express MHCs, and can also kill some virus-infected cells and bacteria such as Salmonella typhi. However, NK cells, which have an excellent effect on killing cancer cells, make up only 5% to 15% of peripheral blood lymphocytes even in normal people, and the percentage drops to less than 1% in patients with severe cancer, so NK cells are limited in their ability to effectively attack cancer cells without further amplification through immunotherapy.

Immunotherapy is a method of extracting the most important immune cells for cancer treatment, such as natural killer cells (NK cells), dendritic cells (DCs), B cells, and T cells, from the patient's blood, cultivating them into immune cells that act strongly against cancer using various types of stimulating substance, and then injecting them back into the patient. Since immunotherapy uses the patient's own blood, immunotherapy has been actively studied in recent years because immunotherapy has fewer side effects and is easier to administer than conventional chemotherapy.

When culturing immune cells, especially when culturing of primarily NK cells is required, the role of T cells, especially help T cells, is necessary, and helper T cells (or Th cells) refer to cells that promote humoral and cellular immunity by regulating the differentiation and activation of white blood cells. They are also called CD4+ T cells because they have the CD4 protein on the cell surface. CD4+ T cells are further classified into Th1, Th2, Th17, and Treg according to their detailed functions. Th1 cells secrete interferon-gamma (IFN-γ) and tumor necrosis factor beta (TNF-β), which induce the fusion of endosomes and lysosomes inside macrophages to form endolysosomes. Meanwhile, Th2 cells secrete various types of interleukins (IL), which cause B cells to differentiate into plasma cells. Th17 cells secrete interleukin-17 (IL-17), which recruits neutrophils. Treg cells, also known as regulatory T cells, do not promote immune responses but rather suppress them, maintaining immune homeostasis and blocking autoimmune responses. Regulatory T cells (Tregs) are components of the immune system that suppress the immune response of other cells, an important "self-check" designed to prevent the immune system from overreacting. Regulatory T cells are involved in stopping the immune response after successfully killing an invading microorganism and are also involved in stopping autoimmune cell diseases. The immune system must be able to distinguish between self and non-self. When self/non-self distinction fails, the immune system destroys cells and tissues in the body, resulting in autoimmune disease. Regulatory T cells actively suppress the activity of the immune system and prevent pathological auto-reactions such as autoimmune diseases. The molecular mechanisms of how regulatory T cells exert their suppressive/regulatory activity are not yet fully understood, and the immunosuppressive cytokines TGF-beta and interleukin 10 (IL-10) have also been implicated in regulatory T cell function.

On the other hand, when different types of antibodies or cytokines are used to activate immune cells, such as regulatory T cells (Tregs) are also activated. However, Tregs take longer to activate than other cells. Therefore, it is not uncommon for cell counts to increase well in the early stages of cell culture, but when cells are cultured for more than about 8 to 9 days, cell counts are not increased as much as expected by activated Tregs. In particular, it is frequently found in cancer patients with low immunity and in patients with a large number of Treg cells and active Treg cells; this phenomenon is conspicuous because of the effect of Treg.

Therefore, there is a need to develop a technology capable of proliferating immune cells in large quantities by minimizing the effects of Tregs.

### Disclosure

### Technical Problem

As a result of research efforts to solve the above problems, the present inventors have completed the present disclosure by developing a technology capable of inhibiting the activity of regulatory T cells (Treg) during immune cell culture.

Therefore, an objective of the present disclosure is to provide an immune cell proliferation activation kit and a method of immune cell culture using the proliferation activation kit, in which one or more immune cells, in particular NK cells and T cells, can be proliferated to a greater extent by inhibiting the activity of CD4+ T cells, in particular regulatory T cells (Treg), at a specific time in immune cell culture.

The objective of the present disclosure is not limited to the objective mentioned above, and even if not explicitly mentioned, an objective of the present disclosure that may be recognized by those skilled in the art from the detailed description of the present disclosure may be naturally included.

### Technical Solution

In order to achieve the objectives of the present disclosure as described above, the present disclosure provides an immune cell proliferation activation kit including a first unit including a T cell stimulating substance; and a second unit including a steroidal agent.

In a preferred embodiment, the first unit is formed such that the T cell stimulating substance is contained in the basic medium solution a concentration of 0.1 to 12 ug/mL when the T cell stimulating substance is an antibody and T cell stimulating substance is contained at a concentration of at least 1×10⁵ cells/mL when the T cell stimulating substance is a feeder cell, in which the antibody is one or more selected from the group consisting of an anti-CD3 antibody, an anti-CD4 antibody, and an anti-CD28 antibody.

In a preferred embodiment, the second unit is formed by including the steroidal agent at a concentration of 0.02 µg/ml or more in a basic medium solution.

In a preferred embodiment, the steroidal agent is glucocorticoids, in which the steroidal agent is at least one selected from the group consisting of cortisol, cortisone, prednisolone, prednisone, methylprednisolone, dexamethasone, betamethasone, triamcinolone, fludrocortisone acetate, and deoxycorticosterone acetate.

In a preferred embodiment, the steroidal agent acts to inhibit regulatory T cell (Treg) activity, thereby causing NK cell proliferation.

A preferred embodiment further includes a third unit, including an NK cell stimulating substance, in which the third unit is formed such that the NK cell stimulating substance is contained in the basic medium solution at a concentration in a range of 0.2 ng/mL to 10 ug/mL.

In a preferred embodiment, NK cells stimulating substance is at least one selected from the group consisting of an anti-CD16 antibody, anti-CD56 antibody, IL-12, IL-15, and IL-18.

In addition, the present disclosure provides a method of culturing immune cells, the method including: a first culturing step in which peripheral blood mononuclear cells (PBMC) isolated from blood are suspended in a basic medium solution and is then cultured for 18 to 72 hours while being treated with the first unit including a T cell stimulating substance; a second culturing step in which the first culture solution having undergone the first culturing step is treated with the second unit including a steroidal agent and incubated for 18 to 25 hours; and a third culturing step in which at least one of the basic medium solution and FBS (FBS, or autologous plasma, the same hereinafter) is added to the second culture solution having undergone the second culturing step, and the second culture solution is cultured at intervals of 24 hours or more from a certain point in time and transferred to a larger culture container as the number of cells increases.

In a preferred embodiment, in the first culturing step, before or simultaneously with the treatment of the first unit, a third unit including an NK cell stimulating substance is treated.

In a preferred embodiment, the third unit is formed such that the NK cell stimulating substance is contained at a concentration of 0.2 ng/mL to 10 ug/mL in a basic medium solution, and the NK cell stimulating substance is at least one selected from the group consisting of anti-CD16 antibodies, anti-CD56 antibodies, IL-12, IL-15, and IL-18.

In a preferred embodiment, the first unit is formed such that T cell stimulating substance is contained in the basic medium solution at a concentration of 0.1 to 12 ug/mL when the T cell stimulating substance is an antibody and T cell stimulating substance is contained at a concentration of at least 1×10⁵ cells/mL when the T cell stimulating substance is a feeder cell, and the antibody is at least one selected from the group consisting of an anti-CD3 antibody, anti-CD4 antibody, and anti-CD28 antibody. The second unit is formed such that a steroidal agent is contained in the basic medium solution at a concentration of 0. 02 µg/ml or more, in which the steroidal agent is a glucocorticoid and is at least one selected from the group consisting of cortisol, cortisone, prednisolone, prednisone, methylprednisolone, dexamethasone, betamethasone, triamcinolone, fludrocortisone acetate, and deoxycorticosterone acetate.

In a preferred embodiment, the first unit is used for the treatment of the PBMC suspended in the basic medium solution on the 0th day of culture, the 1st day of culture, or the 2nd day of culture, and the second unit is used for treatment 20 to 25 hours after the first culture solution is treated with the first unit.

In a preferred embodiment, the second unit is further treated once or more after 5 days of culture in the second culture solution in which the second culturing was performed.

In addition, the disclosure provides a method of culturing immune cells for pets, the method including: a first culturing step in which peripheral blood mononuclear cells (PBMCs) isolated from the blood of a dog are suspended in a basic medium solution and is then cultured for 4 days while sequentially or simultaneously being treated with a third unit including a NK cell stimulating substance and a first unit including a T cell stimulating substance; a second culturing step in which the first culture solution having undergone the first culturing step is simultaneously or sequentially treated with a second unit including a steroidal agent and the third unit and then cultured for 18 to 25 hours; and a third culturing step in which at least one of the basic medium solution and FBS (FBS) is added to the second culture solution having undergone the second culturing, the second culture solution is cultured at intervals of 24 hours or more from a certain point in time and transferred to a larger culture container as the number of cells increases.

In a preferred embodiment, the first unit is formed such that T cell stimulating substance is contained in the basic medium solution at a concentration of 0.1 to 12 ug/mL when the T cell stimulating substance is an antibody and T cell stimulating substance is contained at a concentration of at least 1×10⁵ cells/mL when the T cell stimulating substance is a feeder cell, and the antibody is at least one selected from the group consisting of an anti-CD3 antibody, anti-CD4 antibody, and anti-CD28 antibody. The second unit is formed such that a steroidal agent is contained in the basic medium solution at a concentration of 0.02 ug/ml or more, in which the steroidal agent is a glucocorticoid and is at least one selected from the group consisting of cortisol, cortisone, prednisolone, prednisone, methylprednisolone, dexamethasone, betamethasone, triamcinolone, fludrocortisone acetate, and deoxycorticosterone acetate. The third unit is formed such that the NK cell stimulating substance is contained in the basic medium solution at a concentration of 0.2 ng/mL to 10 ug/mL, in which the NK cell stimulating substance is one or more selected from the group consisting of an anti-CD16 antibody, an anti-CD56 antibody, IL-12, IL-15, and IL-18.

### Advantageous Effects

According to the immune cell culture method using the immune cell proliferation activation kit of the above-described disclosure, the immunosuppressive activity of CD4+ T cells, especially regulatory T cells (Treg), is suppressed by the proliferation activation kit at a certain time during immune cell culture, so that immune cells, especially one or more of NK cells and T cells, can be proliferated with high efficiency of at least two times or more compared to the known immune cell culture method, and also mass proliferation is possible.

These technical effects of the present disclosure are not limited to the above-mentioned scope and may be recognized by those skilled in the art from specific descriptions for implementing the present disclosure, even if not explicitly mentioned.

### Description of Drawings

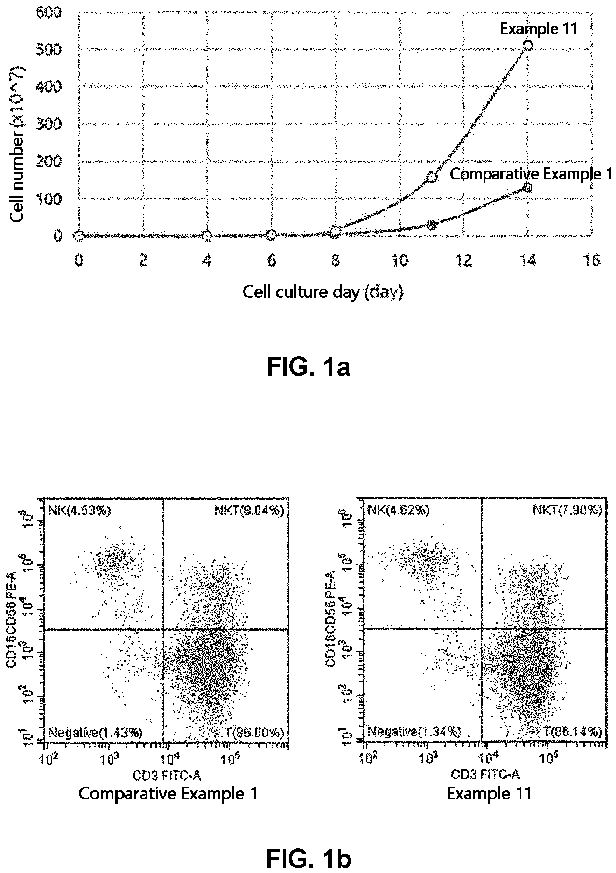

FIG. 1A is a graph showing the change in cell number with incubation time obtained by culturing immune cells according to an immune cell culture method according to one embodiment of the present disclosure and a culture method according to a Comparative Example thereof and FIG. 1B is a graph showing FACS data obtained by flow cytometric analysis of the respective culture media;

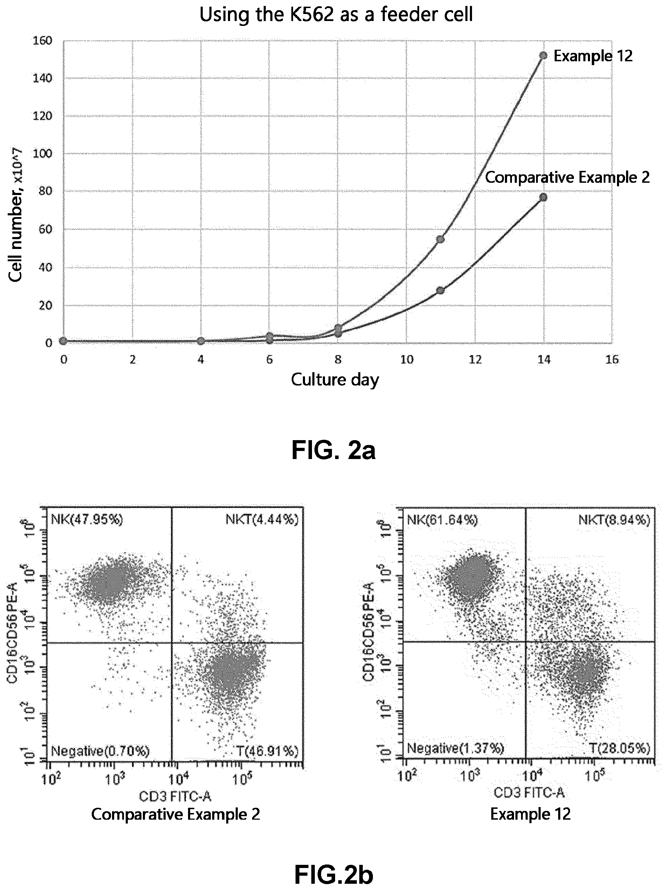

FIG. 2A is a graph showing the change in cell number with culture time obtained by culturing immune cells according to another embodiment of the present disclosure and a culture according to a Comparative Example thereof and FIG. 2B is a graph showing FACS data obtained by flow cytometric analysis of the respective culture media;

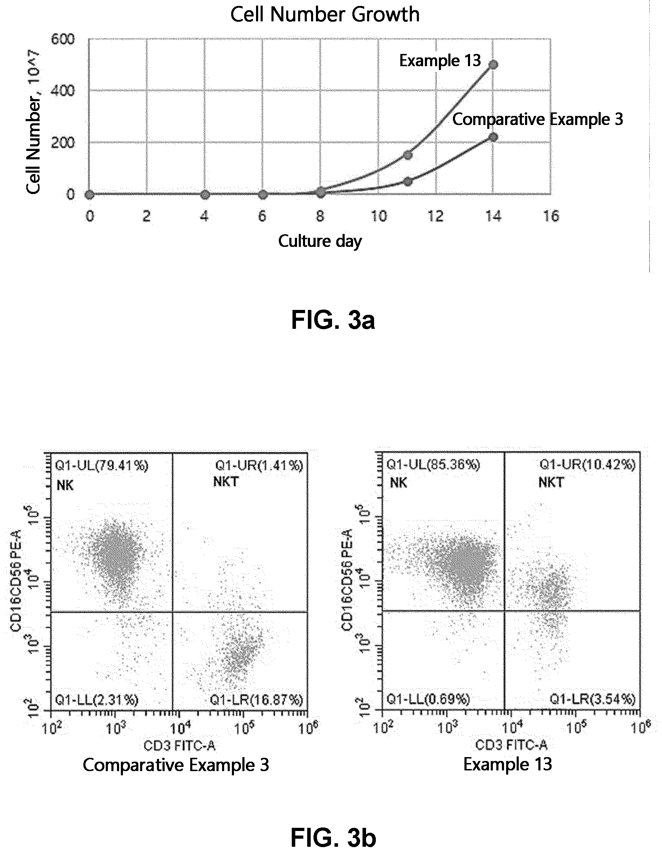

FIG. 3A is a graph showing the change in cell number as a function of culture time obtained by performing cultures with an immune cell culture method according to another embodiment of the present disclosure and a culture method according to a Comparative Example thereof, and FIG. 3B is a graph showing FACS data obtained by flow cytometric analysis of the respective culture media;

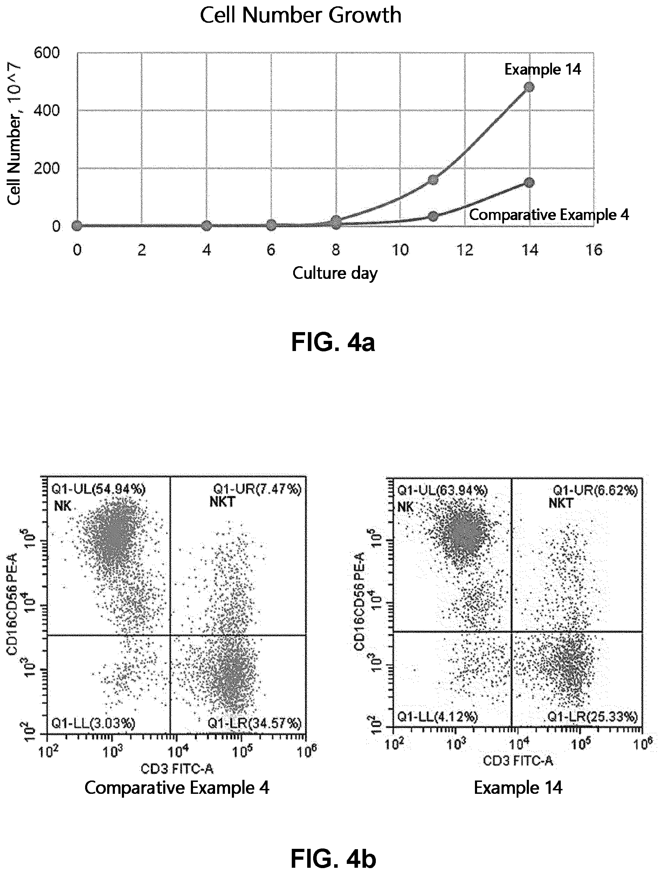

FIG. 4A is a graph showing the change in cell number as a function of culture time obtained by performing cultures with an immune cell culture method according to another embodiment of the present disclosure and a culture method according to a Comparative Example thereof and FIG. 4B is a graph showing FACS data obtained by flow cytometric analysis of the respective culture media;

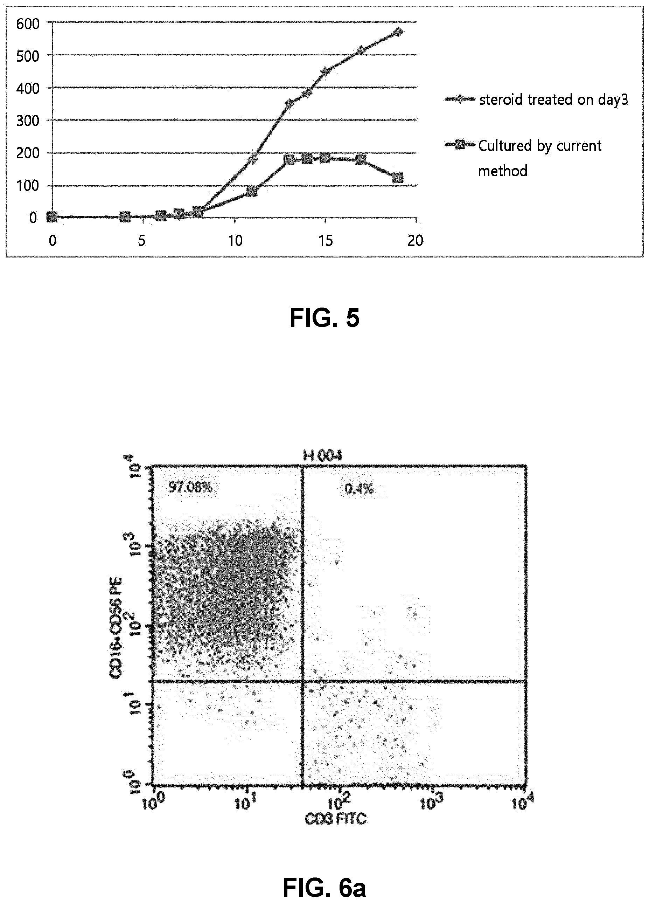

FIG. 5 is a graph showing the change in cell number as a function of culture time obtained by performing cultures with an immune cell culture method according to another embodiment of the present disclosure and a culture method according to a Comparative Example thereof;

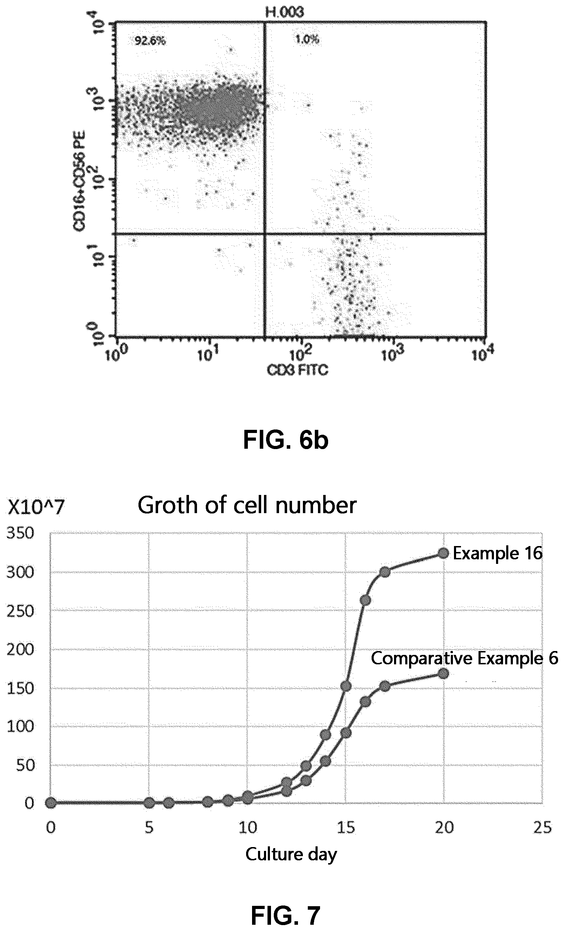

FIG. 6A is a graph showing FACS data of a culture medium cultured with an immune cell culture method according to another embodiment of the present disclosure and FIG. 6B is a graph showing FACS data of a culture medium cultured according to a Comparative Example;
FIG. 7 is a graph showing the change in the number of cells according to the culturing time obtained by culturing with an immune cell culture method according to another embodiment of the present disclosure and a culture method according to a Comparative Example thereof; and

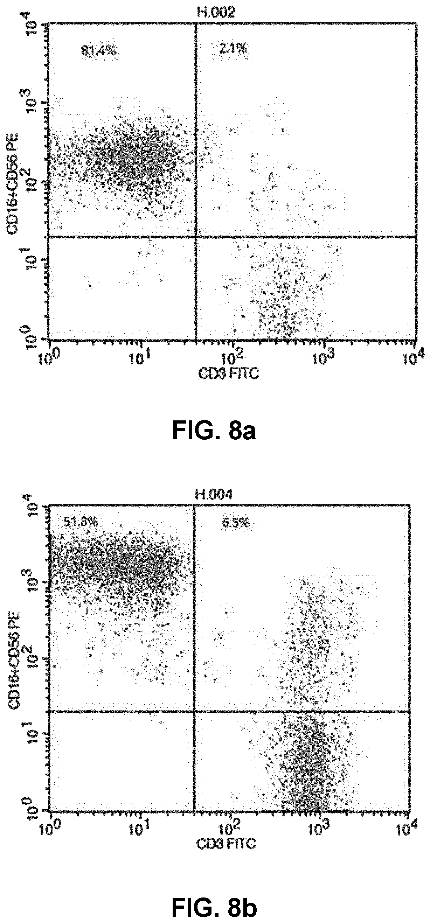

FIGS. 8A and 8B are graphs showing FACS data obtained by culturing treated and untreated cells on day 3, day 4, and day 8, respectively, using cells with an NK cell ratio of approximately 50% and increasing the concentration of steroidal agents included in the immune cell proliferation activation kit by approximately 2 times.

### Best Mode

### Mode for Invention

The terms used herein are used only to describe specific embodiments and are not intended to limit the present disclosure. As used herein, the singular forms "a", "an", and "the" are intended to include the plural forms as well unless the context clearly indicates otherwise. In this specification, the term "include" or "have" should be understood to designate that one or more of the described features, numbers, steps, operations, components, or a combination thereof exist, and the possibility of addition of one or more other features or numbers, operations, components, or combinations thereof should not be excluded in advance.

Terms such as first, second, and the like may be used to describe various components, but the components should not be limited by such terms. The above terms are used only for the purpose of distinguishing one component from another. For example, without departing from the scope of the present disclosure, a first component may be referred to as a second component, and similarly, the second component may also be referred to as a first component.

Unless defined otherwise, all terms used herein, including technical or scientific terms, have the same meaning as commonly understood by those of ordinary skilled in the art to which this disclosure belongs. Terms such as those defined in a generally used dictionary should be interpreted as having meanings consistent with those of the context of related technologies and are not interpreted as ideal or excessively formal meanings unless clearly defined in the present disclosure.

As used herein, the term "immune cell" is used to include only NK cells and T cells. "NK cell" is used to mean both NK cells and NKT cells, but may also be used to mean only NK cells in some cases.

The term "feeder cell" used in the present disclosure is used as an allogeneic cell belonging to a cancer cell line.

As used in the present disclosure, the term "basic medium" refers to a medium including the most basic components of a medium mixed with the nutrients required by the cultured organism to cultivate microorganisms or animal or plant tissues, and commercialized basic media include Basal medium eagle's (BME), minimum essential medium (MEM), Dulbecco's modified eagle's medium (DMEM), and RPMI 440. As used in the present disclosure, "basic medium for culturing suspension cells" refers to a basic medium suitable for culturing suspension cells among the known basic media.

In the case of description of the time relationship, for example, when the time-post relationship is described as "after", "and then", "thereafter", "before", etc., it includes cases where "right" or "direct" is not continuous unless used.

Hereinafter, the technical configuration of the present disclosure will be described in detail with reference to the accompanying drawings and preferred embodiments.

However, the present disclosure is not limited to the embodiments described herein and may be embodied in other forms. Like reference numbers used to describe the disclosure throughout the specification refer to like elements.

The technical features of the present disclosure relate to a novel use of drugs, in particular steroidal agents, for suppressing regulatory T cell (Treg) activity, and in particular to an immune cell proliferation activation kit and a method of immune cell culture using the kit, in which a first unit including a T cell stimulating substance, a second unit including a steroidal agent, and, if necessary, a third unit including an NK cell stimulating substance, are used in a specific sequence and at a specific time in immune cell culture to suppress the immunosuppressive activity of CD4+ T cells, in particular regulatory T cells (Tregs), resulting in a greater and longer proliferation of one or more of NK cells and T cells.

In other words, when culturing immune cells, NK cells need to be stimulated not only at the beginning but also during the culture, while the stimulation of T cells is sufficient for the initial stimulation. Therefore, it is possible to activate more NK cells by stimulating NK cells first with an NK cell stimulating substance, i.e., third unit, and then stimulating T cells with a T cell stimulating substance, i.e., the first unit, so that the activated T cells and the NK cell stimulating substance together stimulate NK cells further, and if the immunosuppressive activity of CD4+ T cells, especially regulatory T cells (Treg), among the activated T cells can be suppressed, NK cells can be activated or proliferated more. This is because it proved the hypothesis that if the first and second units are used sequentially without using the third unit, T cells can be more efficiently proliferated and activated even if more T cells are to be activated or proliferated.

In other words, the present disclosure has developed a new use for steroidal drugs for inhibiting regulatory T cell (Treg) activity by demonstrating that steroidal agents, drugs for inhibiting regulatory T cell (Treg) activity, which is known to suppress CD4+ T cells and, in particular, Treg cells, can be utilized in immune cell cultures at certain stages of culture, immune cells can proliferate and activate more efficiently.

More specifically, steroidal agents are known to inhibit CD4+ T cells such as Th1, Th2, Th17, and Treg from removing inflammation and pain, but they have serious side effects such as immunosuppression when used for a long period of time. In immune cell culture, it was found that inhibition of Treg cells by steroidal agents after NK cell activation did not inhibit NK cell proliferation and activation and that a certain degree of overcrowding of immune cells in culture did not interfere with NK cell activation and cell proliferation. This is because it was found that when Treg cells are inhibited by steroidal agents without stimulating NK cells, steroidal agents proliferate and activate T cells rather than NK cells and that a certain degree of overcrowding of immune cells in culture does not interfere with T cell activation and cell proliferation.

Accordingly, the immune cell proliferation activation kit of the present disclosure includes a first unit including a T cell stimulating substance; and a second unit including a steroidal agent. If necessary, a third unit including a NK cell stimulating substance may be further included.

The first unit includes a T cell stimulating substance, which, when the T cell stimulating substance is an antibody, may be formed by causing the basic medium solution to include the antibody at a concentration of 0.1 to 12 ug/mL, or, when the T cell stimulating substance is a feeder cell, may be formed by causing the basic medium solution to contain at least 1×10⁵ feeder cells/mL, and the antibody used as the T cell stimulating substance may be one or more selected from the group consisting of an anti-CD3 antibody, an anti-CD4 antibody, and an anti-CD28 antibody. In addition, feeder cells are not limited as long as they are of the same species as the immune cells to be cultured; basically, at least 5% of the number of immune cells to be cultured should be used as stimulatory cells, and in many cases up to 100%. Here, the basic medium solution is formed such that interleukin-2 (IL-2) is contained at a concentration of 300 to 4000 IU/mL in the basic medium for suspension cell culture, in which the basic medium for suspension cell culture is used for immune cell culture.

In general, in immune cell culture, NK cells are stimulated to a certain extent by stimulating and activating Th1 by IL-2 included in the basic medium solution, and in this state, if Th cells are stimulated by T cell stimulating substance such as anti-CD3 antibodies to stimulate NK cells and Tc cells by activated Th1 cells, NK cells, and Tc cells are activated, and NK cells are also proliferated to a certain extent. In particular, further stimulation with one or more NK cell stimulating substance selected from the group consisting of IL-12, IL-15, IL-18, anti-CD16 antibodies, and anti-CD56 antibodies included in the third unit prior to treatment with the first unit, as described herein, causes the NK cells to become more activated and grow efficiently.

The second unit includes a steroidal agent and may be formed such that the steroidal agent is contained at a concentration of 0.02 µg/ml or more in the basic medium solution. The steroidal agent included in the second unit may be of the glucocorticoid class, although any steroidal drug known in the art may be used. In one embodiment, the steroidal agent may be at least one selected from the group consisting of cortisol, cortisone, prednisolone, prednisone, methylprednisolone, dexamethasone, betamethasone, triamcinolone, fludrocortisone acetate, and deoxycorticosterone acetate.

As mentioned above, if the first unit is processed in immune cell culture, i.e., if the immune cells are stimulated with T cell stimulating substance such as anti-CD3 antibodies, all T cells are stimulated, especially humoral immune cells such as Th2 and Treg, which are not conducive to NK cell activation and proliferation. In particular, since Treg cells interfere with the activation and proliferation of NK cells, T cells can be proliferated better than NK cells among immune cells if the second unit is treated with the first culture solution. NK cells can be proliferated better than T cells by neutralizing Treg cells by treating the third unit first and then treating the second unit in the first culture solution treated with the first unit.

Here, the steroidal agent included in the second unit acts on the proliferation of immune cells, especially NK cells, by inhibiting the immunosuppressive activity of CD4+ T cells, especially regulatory T cells (Treg), and experimentally, the steroidal agent has been shown to eliminate the immune response inhibitory activity of regulatory T cells, thereby inhibiting the growth of T cells in general to some extent.

In other words, it was confirmed that steroidal agents, which are drugs for suppressing the activity of regulatory T cells (Treg), do not affect the proliferation of cellular immune cells, such as once activated NK cells and Tc cells, at all, as shown in the experimental examples described later. This is because it was confirmed that the NK cell ratio increased as the culture time increased. In particular, when the concentration of the steroidal agent is increased, the proliferation rate of T cells is suppressed to some extent, and the NK cell ratio is relatively increased in proportion to the steroidal agent concentration.

When the content of the steroidal agent included in the second unit is less than 0.02 ug/ml, the properties of suppressing the immunosuppressive activity of CD4+ T cells, especially regulatory T cells (Treg), are not sufficiently exhibited, and even if the steroidal agents are treated at a high concentration, it was predicted that the effect on immune cell culture would not change significantly when the concentration exceeded 9 ug/ml. Experimentally, considering that even if the culture contains more than 20µg/ml, there is no greater progress or adverse effect on the immune cell proliferation effect, the upper limit of the steroid content can be determined in consideration of economic feasibility. Therefore, the steroidal agents used as a drug for suppressing Treg activity in the second unit of the kit for activating immune cell proliferation of the present disclosure can obtain desired effects as long as it is included at a concentration of 0.02 ug/ml or more.

The third unit includes an NK cell stimulating substance, which may be formed such that the NK cell stimulating substance is contained in the basic medium solution at a concentration of 0.2 ng/mL to 10 ug/mL, in which the NK cell stimulating substance may be one or more selected from the group consisting of an anti-CD16 antibody, an anti-CD56 antibody, IL-12, IL-15, and IL-18. Here, the concentration of the NK cell stimulating substance included in the third unit varies depending on the type of NK cell stimulating substance, in which the third unit may be prepared by adding IL-12 and IL-18 to a basic medium solution and then dissolving the mixed solution, such that IL-12 is included at a concentration of 0.5-15 ng/mL and IL-18 is included at a concentration of 2-50 ng/mL, in a first embodiment, or by adding IL-15 alone to a basic medium solution and then dissolving the mixed solution, such that IL-15 is included at a concentration of 1-100 ng/mL, in a second embodiment. As another embodiment, the third embodiment, the third unit can be prepared to include at least one of an anti-CD16 antibody at a concentration of 0.1 to 10 ug/mL and an anti-CD56 antibody at a concentration of 0.1 to 10 ug/mL in the basic medium solution. It will be appreciated that the first embodiment may further include one or more anti-CD16 antibody and an anti-CD56 antibody to implement the third unit if desired.

As described above, when Th1 is stimulated and activated by IL-2 in immune cell culture using the immune cell proliferation activation kit of the present disclosure, and NK cells are further stimulated by NK cell stimulating substance such as anti-CD16 antibody and anti-CD56 antibody, NK cells can be proliferated and activated more efficiently than T cells for long-term culture, and T cells can be proliferated and activated more efficiently than NK cells for long-term culture when the third unit is not used because the basic medium solution includes IL-2.

The immune cell proliferation activation kit of the present disclosure having such a configuration may include a first unit, a second unit, and/or a third unit, each individually packaged and having different contents, i.e., different components, to be added to the medium at each step of the immune cell culture method of the present disclosure described herein. In addition, since NK cells in immune cell culture require the help of stimulated T cells to be fully activated after initial stimulation, the proliferation activation kit of the present disclosure requires the first unit to be used and the second unit to be used after the T cells in the immune cell culture medium are stimulated. In this way, when the immune cell proliferation activation kit of the present disclosure is used sequentially in immune cell culture, that is, when the first unit is treated to stimulate T cells in the immune cell culture medium and the second unit is treated at the appropriate time, the proliferation rate of T cells among immune cells can be increased very effectively when the third unit is not used, and the proliferation rate of NK cells among immune cells can be increased when the third unit is used before the first unit is used.

In addition, the immune cell culture method of the present disclosure includes: a first culturing step in which peripheral blood mononuclear cells (PBMC) isolated from blood are suspended in a basic medium solution and is then cultured for 18 to 72 hours while being treated with a first unit including a T cell stimulating substance; a second culturing step in which the first culture solution having undergone the first culturing step is treated with a second unit including a steroidal agent and incubated for 18 to 25 hours; and a third culturing step in which at least one of the basic medium solution and FBS (FBS) is added to the second culture solution having undergone the second culturing step, the second culture solution is cultured at intervals of 18 to 25 hours and transferred to a larger culture container as the number of cells increases.

A third unit including an NK cell stimulating substance may be processed before or simultaneously as the first unit in the first culturing step. In other words, as described above, NK cells are stimulated to some extent by the inclusion of IL-2 in the basic medium solution, but when the first unit is treated and then the second unit is treated with one or more NK cell stimulating substance selected from the group consisting of an anti-CD16 antibody, an anti-CD56 antibody, IL-12, IL-15, and IL-18, the NK cells among the immune cells may grow better, so it may be desirable to treat the third unit before treating the first unit when it is desired to increase the proportion of NK cells among the immune cells.

The second culturing step is performed by culturing the second unit for a period of time after treating the first unit in the first culture solution having undergone the first culturing step, so that the second culturing step may be performed 18 to 48 hours after the treatment of the first unit.

In one embodiment, the second culturing can be performed by treating the second unit with the first culture solution formed by treating the first unit at any point during day 0, day 1, or day 2 of culture with the PBMC-suspended basic medium solution and culturing for 20 to 25 hours. In other words, on day 0 of immune cell culture, NK cells are stimulated to some extent by IL-2 included in the basic medium solution, and then on day 0, 1, or 2 of culture, T cells are stimulated with T cell stimulating substance such as an anti-CD3 antibody, anti-CD4 antibody, or anti-CD28 included in the first unit to activate Tc, NK cells, etc. Subsequent treatment with the second unit including a steroidal agent on any one of days 1 to 4 of the culture, inhibits the immunosuppressive activity of CD4+ T cells, in particular regulatory T cells (Treg), so that immune cells, including NK cells, proliferate and/or are activated because the suppressive activity of regulatory T cells (Treg) is prevented.

The third culturing is a process for harvesting immune cells by culturing immune cells in large quantities, and the second culture solution in which the second culturing was performed is cultured by adding one or more of the basic medium solution and fetal bovine serum (FBS) at intervals of 24 hours or more from a predetermined time and may be transferred to a larger culture container as the immune cells proliferate, and the number of cells increases while adding at intervals of 48 hours or more, as in the embodiment described below. If necessary, the second unit may be further processed in the third culturing, and as one embodiment, the second culture solution in which the second culturing has been performed may be further processed once or more after 5 days of culture. As is known, PBMCs proliferate well in the early stage of culture, that is, when the immune cells are young (early stage of culture), but after about 8 to 10 days of culture, the proliferation rate decreases, and they do not grow well, but in the present disclosure, the third stage of culture can be performed for as short as 11 days and as long as 21 days, so a large amount of NK cells can be harvested compared to the conventional immune cell culture method, which must be performed for as long as 8 days.

This prolongation of the third culturing is based on the experimental results of the present disclosure, which show that when immune cells are stimulated with the basic medium solution at the beginning of immune cell culture, i.e., the first culturing step, and when T cells are further stimulated with the first unit and then treated with the second unit including a steroidal agent to suppress Treg, immune cells are activated and proliferate relatively well by increasing the concentration of cytokines including IL-2 even after 8 to 10 days of culture. Considering the number of cells proliferated in the second culture solution in which the second culturing is performed, the process can be performed by adding one or more of the basic medium solutions and FBS in the required number and content at intervals of 18 to 25 hours from a predetermined time point, as in the embodiment described later. Here, the predetermined time point may be a time point after about 24 hours or more from the time point at which the second unit is added in the second culturing.

Next, the pet immune cell culture method of the present disclosure includes: a first culturing step in which peripheral blood mononuclear cells (PBMCs) isolated from the blood of a dog are suspended in a basic medium solution and is then cultured for 4 days while simultaneously or sequentially being treated with the first unit including a T cell stimulating substance and a third unit including a NK-cell stimulating substance; a second culturing step in which the first culture solution having undergone the first culturing step is treated with a second unit including a steroidal agent and the third unit simultaneously or sequentially and is then cultured for 18 to 25 hours; and a third culturing step in which at least one of the basic medium solution and FBS (FBS) is added to the second culture solution having undergone the second culturing step at intervals of 24 hours or more from a certain point in time and the second culture solution is transferred to a larger culture container as the number of cells increases.

The pet immune cell culture method of the present disclosure is a method for massively proliferating immune cells, including NK cells, from PBMCs of animals rather than PBMCs of humans and is broadly similar to the immune cell culture method described above in that the culturing utilizes the immune cell proliferation activation kit of the present disclosure. However, in order to more efficiently proliferate immune cells with PBMCs isolated from pets, especially dogs, rather than human blood, when performing the first culturing step to the third culturing step, it differs only in whether two or more units of the first unit or the third unit are used simultaneously and the time of processing, and the rest of the composition is the same, so a detailed description will be omitted.

However, it was experimentally confirmed that in the pet immune cell culture method, the effect is more excellent only when IL-15 is included in the third unit.

### Example 1

An immune cell proliferation activation kit 1 was prepared by preparing the first to third units as follows.

### 1. Preparation of basic medium solution

Basic medium for suspension cell culture (trade name: RPMI 1640) to which IL-2 was added to a concentration of 2000 IU/mL and then dissolved to prepare the basic medium solution.

### 2. First unit preparation

First unit-1 was prepared by adding and dissolving anti-CD3 antibody to the basic medium solution to a concentration of 1 ug/mL.

### 3. Second unit preparation

Second unit-1 was prepared by dissolving dexamethasone sodium phosphate in a basic medium solution at a concentration of 0.02ug/mL.

### Example 2

An immune cell proliferation activation kit 2 was prepared by performing the same method as in Example 1, except that in the preparation of the second unit, dexamethasone sodium phosphate was dissolved to include a concentration of 20 ug/mL in the basic medium solution to prepare the second unit-2.

### Example 3

An immune cell proliferation activation kit 3 was prepared by performing the same method as in Example 1, except that in the preparation of the second unit, dexamethasone sodium phosphate was dissolved to include a concentration of 1 ug/mL in the basic medium solution to prepare the second unit-3.

### Example 4

An immune cell proliferation activation kit 4 was prepared by performing the same method as in Example 1, except that in the preparation of the second unit, prednisolone (Sorondo, 5mg/tablet, Yuhan Yanghaeng) was dissolved in the basic medium solution to a concentration of 167 ug/mL, and then diluted 20 times with the basic medium solution to a concentration of 8.3 ug/mL to prepare the second unit-4.

### Example 5

An immune cell proliferation activation kit 5 was prepared by performing the same method as in Example 1, except that in the preparation of the second unit, methylprednisolone (PD tablet, 4 mg/tablet, Jungwoo Pharmaceutical) was dissolved in the basic medium solution to a concentration of 80 ug/mL, and then diluted 10 times with the basic medium solution to a concentration of 8 ug/mL to prepare the second unit-5.

### Example 6

An immune cell proliferation activation kit 6 was prepared by performing the same method as in Example 1, except that in the preparation of the second unit, 1 uL of betamethasone sodium phosphate (Hanol Betamethasone Ju, 5.2 mg/1 mL ampule, Hanol Biopharma) was dissolved in 10 mL of the basic medium solution to have a concentration of 0.4 ug/mL to prepare the second unit-6.

### Example 7

An immune cell proliferation activation kit 7 was prepared by performing the same method as in Example 1, except that in the preparation of first unit, 5×10⁶ K562 cell lines were dissolved per 1 ml of the basic medium solution instead of anti-CD3 antibody to prepare the first unit-2.

### Example 8

An immune cell proliferation activation kit 8 was prepared in the same manner as in Example 1, except that the third unit was further prepared as follows.

IL-12 and IL-18 were added to the basic medium solution to include IL-12 at a concentration of 10 ng/mL and IL-18 at a concentration of 50 ng/mL and then dissolved to prepare the third unit-1.

### Example 9

An immune cell proliferation activation kit 9 was prepared by performing the same method as in Example 7, except that in the preparation of the third unit, anti-CD16 and anti-CD56 were added to the basic medium solution to include a concentration of 1 ug/mL each, and then dissolved to prepare the third unit-2.

### Example 10

An immune cell proliferation activation kit 10 was prepared by performing the same method as in Example 7, except that in the preparation of the third unit, IL-15 was added to the basic medium solution to include IL-15 at a concentration of 20 ng/mL and then dissolved to prepare the third unit-3.

### Experiment method

### 1. Cell counting

Cell counts were measured on specific culture days, with the first day PBMCs were isolated from blood and cultured being Day0 (1×10⁷ cells) and cultured for a total of 14 or 19 days. Collect both cells and medium in a 500 ml conical tube and centrifuge at 1,800 rpm for 5 minutes at 4°C, then remove the supernatant. Cells were suspended by adding 10 ml of DPBS to the cell pellet, 10 µg of which was taken and diluted, mixed with an equal amount of 0.4% Trypan blue staining solution (Gibco, NY, USA), and placed on a C-Chip (iNCYTO, GA, USA) to count the number of cells under a culture microscope CKX53 (Olympus, Tokyo, Japan).

### 2. Flow cytometric analysis

The cultured cells were centrifuged at 2,000 rpm for 3 min at 4°C to remove the supernatant, resuspended in 200 µl of FCS buffer (eBioscienceTMFlow cytometry staining buffer, Invitrogen), and centrifuged at 2,000 rpm for 3 min at 4°C. The supernatant was removed, and 5 µl of each antibody (anti-human CD56-PE, anti-human CD16 PE-Cy-7, anti-human CD3-FITC) and isotype control antibody (Mouse IgG1 kappa isotype control-PE, Mouse IgG1 kappa isotype control-FITC, Mouse IgG1 kappa isotype control PE-Cy-7) were added and reacted under dark conditions and at 2°C to 4°C. After 30 minutes, centrifugation was performed at 2,000 rpm at 4°C for 3 minutes, and the supernatant was removed. After resuspension with 500 ul FCS buffer, centrifugation was performed at 2,000 rpm at 4°C for 3 minutes, and the supernatant was removed. After washing the total cells twice, the cells were suspended in 500 µl of FCS buffer and analyzed by FACSCanto II (Becton Dickinson).

### Example 11

Immune cells were cultured using the immune cell proliferation activation kit 1 in the following manner. During immune cell culture, cells were counted, and flow cytometric analysis was performed using the experimental methods described above, and the results are shown in Table 1 and FIGS. 1A and 1B.

### 1. Isolation of human peripheral blood mononuclear cells (PBMC)

① Put 15 ml of Ficoll-PaqueTM PLUS (GE Healthcare, SWEDEN) in a 50 mL Leukosep tube (Greiner Bio-One) and centrifuge at 1,200 rpm at 4°C for 3 minutes.
② Based on 30 cc of blood collected in a BD vacutainer (BD vacutainer, NJ, USA), add 30 cc blood to one tube of ① and centrifuge at 2,000 rpm at 4°C for 20 minutes.
③ After centrifugation, transfer the plasma corresponding to the uppermost layer to a new 50 mL conical tube, heat the resultant three times for 10 minutes in a 56°C water bath, and centrifuge at 1,500 rpm at 4°C for 5 minutes and label the supernatant as Heat-Inactivated Plasma (HIP) and store at 4°C.
④ After separating plasma, transfer the remaining peripheral blood mononuclear cell (PBMC) layer to a new 50 mL conical tube and centrifuge at 1,500 rpm at 4°C for 5 minutes.
⑤ Remove the supernatant, wash the cells with 20 mL of DPBS (Dulbecco's Phosphate-Buffered Saline, Welgene, KOREA), and centrifuge at 1,200 rpm at 4°C for 5 minutes.
⑥ Remove the supernatant, suspend the cells in 5 mL DPBS, dilute 10 µl of suspended cells with 0.4% Trypan blue staining solution (Gibco, NY, USA) in the same amount, and insert the 10 µl of diluted cell solution into a C-Chip (iNCYTO, GA, USA) and count the number of cells under a culture microscope CKX53 (Olympus, JAPAN) . As a result, the number is found to be 1×10⁷ cells per 1 mL of blood.

### 2. Immune cell culture

### ① Day 0 (Day 0): Day of PBMC isolation from blood.

PBMCs (1×10⁷ cells) were suspended in 5 mL of basic medium solution and placed in T25 cell culture flasks (SPL, KOREA: example flask), the anti-CD3 antibody was added to a concentration of 1 ug/mL, and the cells were cultured for 24 hours at 37°C and 5% CO₂ culture conditions. In order to use as a control group, cells were cultured in the same conditions in another flask as in Comparative Example 1.

### ② Day 1: Add a second unit

After adding the second unit-1 so that only the example flask contained 0.2 ug/mL of dexamethasone sodium phosphate, the culture was continued under the same conditions.

### ③ Day 4 to Day 14: Add basic medium solution and FBS

While adding 25 ml of basic medium solution and 2.5 ml of FBS (or autologous plasma) to both the example flask and the control flask, the flask was transferred to T75. Thereafter, as the number of cells increased under the conditions shown in Table 1 below, the cells were transferred to a large flask and cultured for a total of 2 weeks.

**[Table 1]**

| | Culture day | Day0 | Day1 | Day4 | Day6 | Day8 | Day11 | Day14 |
|---|---|---|---|---|---|---|---|---|
| Comparative Example 1 (Control group) | Basic medium solution (mL) | 5 | | 25 | 70 | 300 | 300 | |
| | First unit-1 | Adding | | | | | | |
| | FBS (mL) | 0.5 | | 2.5 | 5 | 10 | | |
| | Cell number (1 × 10⁷) | 1 | | 0.55 | 1.5 | 6 | 32 | 132 |
| Example 11 | Basic medium solution (mL) | 5 | | 25 | 70 | 300 | 300 | |
| | First unit-1 | Adding | | | | | | |
| | Second unit-1 | | Adding | | | | | |
| | FBS (mL) | 0.5 | | 2.5 | 5 | 10 | | |
| | Cell number (1 × 10⁷) | 1 | | 0.85 | 4.1 | 18 | 160 | 512 |
| Culture container (Flask & bag) | | T25 | T25 | T75 | T150 | bag | bag | bag |

As shown in Table 1 and FIG. 1A, culture results show that in Example 11, in which immune cells are stimulated with the anti-CD3 antibody included in the first unit-1 and then cultured with the steroidal agent included in the second unit-1 in the cell culture medium one day later, the cells proliferate better than in Comparative Example 1, in which the second unit-1 is not treated. In particular, it was confirmed that cells were increased significantly after 8 days of culture and multiplied by as much as 5 times or at least 4 times. Furthermore, flow cytometric analysis of the cultured immune cells showed that both Example 11 and Comparative Example 1 proliferated more T cells than NK cells, as shown in FIG. 1B, because NK cells were not stimulated in large numbers before treatment with the first unit. In the case of Example 11 in which the second unit was treated, relatively more NK cells were proliferated compared to Comparative Example 1, in which the second unit was not treated at all, and it can be seen that the second unit increased the NK cell proliferation rate under the same culture conditions.

### Example 12

Human PBMCs were prepared in the same manner as in Example 11 and immune cells were cultured using the immune cell proliferation activation kit 7 as described below. During immune cell culture, cells were counted, and flow cytometric analysis was performed using the experimental methods described above, and the results are shown in Table 2 and FIGS. 2A and 2B.

### ① Day 0: Day of PBMC isolation from blood

PBMCs (1×10⁷ cells) were suspended in 5 mL of basic medium solution and placed in T25 cell culture flasks (SPL, KOREA: example flask), and cultured the cells for 48 hours at 37°C and 5% CO₂ culture conditions. In order to use as a control group, cells were cultured in the same conditions in another flask as in Comparative Example 2.

### ② Day 2: Add a first unit

Both the example flask and the control flask contained 5×10⁶ of the feeder cell, K562 cells, and continued to be cultured under the same conditions after the addition of the first unit-2.

### ③ Day 4: Add a second unit, basic medium solution, and FBS

While adding 25 ml of the basic medium solution and 2.5 ml of FBS to both the example flask and the control flask, the flask was transferred to T75. In particular, the second unit-2 was added to the example flask to contain 20ug/mL of dexamethasone sodium phosphate, unlike the control flask. Example flasks and control flasks were continuously cultured under the same conditions.

### ③ Day 5 to Day 14: Add basic medium solution and FBS

As the number of cells increased under the conditions shown in Table 2 below, they were transferred to a large flask and cultured for a total of 2 weeks.

**[Table 2]**

| | Culture day | Day0 | Day2 | Day4 | Day6 | Day8 | Day11 | Day14 |
|---|---|---|---|---|---|---|---|---|
| Comparative Example 2 (Control group) | Basic medium solution (mL) | 5 | | 25 | 70 | 300 | 300 | |
| | First unit-2 | | Adding | | | | | |
| | FBS (mL) | 0.5 | | 2.5 | 5 | 10 | | |
| | Cell number (1 × 10⁷) | 1 | | 1.1 | 1.4 | 5.2 | 27.5 | 77 |
| Example 12 | Basic medium solution (mL) | 5 | | 25 | 70 | 300 | 300 | |
| | First unit-2 | | Adding | | | | | |
| | Second unit-2 | | | Adding | | | | |
| | FBS (mL) | 0.5 | | 2.5 | 5 | 10 | | |
| | Cell number (1 × 10⁷) | 1 | | 1.1 | 3.8 | 8.2 | 55 | 152 |
| Culture container (Flask & bag) | | T25 | T25 | T75 | T150 | bag | bag | bag |

As shown in Table 2 and FIG. 2A, the culture results show that Example 12, in which the steroidal agent included in the second unit-2 is added to the cell culture medium one day after stimulation of the immune cells with the K562 cell line, which is a feeder cell included in the first unit-2, the cells proliferate better than in Comparative Example 1, in which the second unit-1 is not treated. In particular, it can be seen that the proliferation is significantly increased from the 8th day of culture and is at least 2 times. Furthermore, flow cytometric analysis of the cultured immune cells showed that NK cells proliferated more than T cells in both Example 12 and Comparative Example 2, even though the third unit was not treated before the first unit treatment, as shown in FIG. 2B. In particular, when looking at the ratio of proliferated NK cells to T cells in Example 12, where the second unit is treated, and in Comparison 2, where the second unit is not treated at all, it can be seen that in Example 12, NK cells proliferate more than twice as much as T cells, but in Comparative Example 2, the proliferation ratio of NK cells and T cells is similar, indicating that the second unit increases the proliferation rate of NK cells more than T cells under the same culture conditions. In particular, when feeder cells are used as T cell stimulating substance, it can be seen that NK cell proliferation rate increases even if the third unit is not used before the first unit treatment.

### Example 13

Human PBMCs were prepared in the same manner as in Example 11, and immune cells were cultured using the immune cell proliferation activation kit 8 as described below. During immune cell culture, cells were counted, and flow cytometric analysis was performed using the experimental methods described above, and the results are shown in Table 3 and FIGS. 3A and 3B.

### ① Day 0: Day of PBMC isolation from blood.

PBMCs (0.5×10⁷ cells) were suspended in 5 mL of basic medium solution and placed in a T25 cell culture flask (SPL, KOREA: example flask), IL-12 was present at a concentration of 10 ng/mL and IL-18 was present at 50 ng/mL, and then the third unit-1 was added, and the cells were cultured for 24 hours at 37°C under 5% CO₂ culture conditions. In order to use as a control group, cells were cultured in the same conditions in another flask as in Comparative Example 3.

### ② Day 1: Add a first unit

The cells were cultured for 24 hours at 37°C under 5% CO₂ culture conditions after the first unit-1 was added so that anti-CD3 antibodies were present in both the example flask and the control flask at a concentration of 1 ug/mL.

### ② Day 2: Add a second unit

After adding the second unit-1 so that only the example flask contained 0.2 ug/mL of dexamethasone sodium phosphate, the culture was continued under the same conditions.

### ③ Day 4 to Day 14: Add basic medium solution and FBS

While adding 25 ml of basic medium solution and 2.5 ml of FBS to both the example flask and the control flask, the flask was transferred to T75. Thereafter, as the number of cells increased under the conditions shown in Table 3 below, the cells were transferred to a large flask and cultured for a total of 2 weeks.

As shown in Table 3 and FIG. 3A, the culture results show that Example 13 in which the NK cell stimulating substance IL-12 and IL-18 included in the third unit-1 were first treated, immune cells were stimulated with anti-CD3 antibodies included in the first unit, and then steroidal agent included in the second unit-1 was placed in a cell culture medium a day later, showed better cell proliferation than Comparative Example 3, in which the second unit-1 was not treated. In particular, it can be seen that after the 8th day of culture, the proliferation is significantly increased and multiplied at least twice.

**[Table 3]**

| | Culture day | Day0 | Day1 | Day2 | Day 4 | Day 6 | Day 8 | Day11 | Day14 |
|---|---|---|---|---|---|---|---|---|---|
| Comparative Example 3 (Control group) | Basic medium solution (mL) | 5 | | | 25 | 70 | 300 | 300 | |
| | Third unit-1 | Adding | | | | | | | |
| | First unit-1 | | Adding | | | | | | |
| | FBS (mL) | 0.5 | | | 2.5 | 5 | 10 | | |
| | Cell number (1 × 10⁷) | 0.5 | | | 0.55 | 1.4 | 6.5 | 52 | 225 |
| Example 13 | Basic medium solution (mL) | 5 | | | 25 | 70 | 300 | 300 | |
| | Third unit-1 | Adding | | | | | | | |
| | First unit-1 | | Adding | | | | | | |
| | Second unit-1 | | | Addin g | | | | | |
| | FBS (mL) | 0.5 | | | 2.5 | 5 | 10 | | |
| | Cell number (1 × 10⁷) | 0.5 | | | 0.6 | 2.2 | 17 | 157 | 505 |
| Culture container (Flask & bag) | | T25 | T25 | | T75 | T150 | bag | bag | bag |

Furthermore, flow cytometric analysis of cultured immune cells showed that both Example 13 and Comparative Example 3 proliferated more NK cells than T cells as a result of stimulation of NK cells by treatment with the third unit before treatment with the first unit, as shown in FIG. 3B. In particular, when looking at the ratio of proliferated NK cells to T cells in Example 13, in which the second unit was treated, and in Comparison 3, in which the second unit was not treated at all, it can be seen that in Example 13, NK cells proliferated almost 30 times more than T cells, but in Comparative Example 3, NK cells proliferated about 4.5 times more than T cells, indicating that the second unit significantly increased the proliferation rate of NK cells over T cells in the same culture conditions. In particular, when a third unit-1 including IL-12 and IL-18 is used before the first unit treatment, the proportion of T cells among very effectively cultured immune cells is very small, and NK cells can be proliferated to account for almost the majority.

### Example 14

Human PBMCs were prepared in the same manner as in Example 11, and immune cells were cultured using the immune cell proliferation activation kit 9 as described below. During immune cell culture, cells were counted, and flow cytometric analysis was performed using the experimental methods described above, and the results are shown in Table 4 and FIGS. 4A and 4B.

### ① Day 0: Day of PBMC isolation from blood.

PBMCs (0.5×10⁷ cells) were suspended in 5 mL of basic medium and placed in T25 cell culture flasks (SPL, KOREA: example flasks), anti-CD16 and anti-CD56 antibodies were added at a concentration of 1 ug/mL each, and the cells were cultured for 24 hours at 37°C under 5% CO₂ culture conditions. In order to use as a control group, cells were cultured in the same conditions in another flask as in Comparative Example 4.

### (2) Day 1: Add a first unit

The cells were cultured for 24 hours at 37°C under 5% CO₂ culture conditions after the first unit-1 was added so that anti-CD3 antibodies were present in both the example flask and the control flask at a concentration of 1 ug/mL.

### ② Day 2: Add a second unit

After adding the second unit-2 so that only the example flask contained 20 ug/mL of dexamethasone sodium phosphate, the culture was continued under the same conditions.

### ③ Day 4 to Day 14: Add basic medium solution and FBS

While adding 25 ml of basic medium solution and 2.5 ml of FBS to both the example flask and the control flask, the flask was transferred to T75. Thereafter, as the number of cells increased under the conditions shown in Table 4, the cells were transferred to a large flask and cultured for a total of 2 weeks.

As shown in Table 4 and FIG. 4A, the culture results show that Example 14, in which the immune cells are first treated with anti-CD16 antibody and anti-CD56 antibody, which are NK cell stimulating substance included in the third unit-2, and then stimulated with anti-CD3 antibody included in the first unit-1, and then cultured with the steroidal agent included in the second unit-1 in the cell culture medium one day later, has better cell proliferation than Comparative Example 4, in which the second unit-1 is not treated. In particular, it can be seen that after the 8th day of culture, the proliferation is significantly increased and multiplied at least twice.

**[Table 4]**

| | Culture day | Day0 | Day1 | Day2 | Day 4 | Day 6 | Day 8 | Day11 | Day14 |
|---|---|---|---|---|---|---|---|---|---|
| Comparative Example 4 (Control group)) | Basic medium solution (mL) | 5 | | | 25 | 70 | 300 | 300 | |
| | Third unit-2 | Adding | | | | | | | |
| | First unit-1 | | Adding | | | | | | |
| | FBS (mL) | 0.5 | | | 2.5 | 5 | 10 | | |
| | Cell number (1 × 10⁷) | 0.5 | | | 0.55 | 1.4 | 6.5 | 52 | 225 |
| Example 14 | Basic medium solution (mL) | 5 | | | 25 | 70 | 300 | 300 | |
| | Third unit-2 | Adding | | | | | | | |
| | First unit-1 | | Adding | | | | | | |
| | Second unit-2 | | | Adding | | | | | |
| | FBS (mL) | 0.5 | | | 2.5 | 5 | 10 | | |
| | Cell number (1 × 10⁷) | 0.5 | | | 0.6 | 2.2 | 17 | 157 | 505 |
| Culture container (Flask & bag) | | T25 | T25 | | T75 | T150 | bag | bag | bag |

Furthermore, flow cytometric analysis of cultured immune cells showed that both Example 14 and Comparative Example 4 proliferated more NK cells than T cells as a result of stimulation of NK cells by treatment with the third unit before treatment with the first unit, as shown in FIG. 4B. In particular, when looking at the ratio of proliferated NK cells to T cells in Example 14, in which the second unit was treated, and in Comparison 3, in which the second unit was not treated at all, it can be seen that in Example 14, NK cells proliferated almost 2.5 times more than T cells, but in Comparative Example 3, NK cells proliferated about 1.5 times more than T cells, indicating that the second unit significantly increased the proliferation rate of NK cells over T cells in the same culture conditions. However, the ratio of proliferated NK cells to T cells is not high when compared to Example 13, in which the third unit-1, including IL-12 and IL-18, was used before the first unit treatment, indicating that the IL-12 and IL-18 included in the third unit-1 work more effectively as NK cell stimulating substance than the anti-CD16 and anti-CD56 antibodies included in the third unit-2.

### Example 15

The immune cell culture process was performed as follows, using not the basic medium solution but the medium addition kit for immune cell culture (NKTM) disclosed in the related patent and the immune cell proliferation activation kits 3 to 6 of the present disclosure.

In the other hand, a medium addition kit for immune cell culture (NKTM) is composed of B unit (basic solution, B solution), C1-1 unit (cytokine 1-1 solution), C1-2 unit, C2 unit, A1 unit, A2 unit, and D unit, each individually packaged and including different components to be added to the medium at each stage of immune cell culture. Since a detailed description of each unit and a detailed description of the specific conditions for the culture process of culturing immune cells using the unit (NKTM culture process) are disclosed in Patent Publication No. 10-2018-0057359 (hereinafter referred to as the "prior patent"), only on the parts that differ from the parts described in the prior patent will be focused. However, since the components included in the first unit and third unit including the immune cell proliferation activation kit of the present disclosure are included in each unit including the NKTM, the test was performed using only the second unit-3 to second unit-6 without using the first unit and third unit separately.

### 1. Preparation of components of the medium addition kit (NKTM) for immune cell culture

### ① Preparation of basic solution (solution B)

The basic solution (B-solution) was prepared by dissolving 2.2 mg of IL-2 and 100 mL of 500 mM L-glutamine solution in the basic medium for suspension cell culture (when IL-2 or L-glutamine is already present in the basic medium, adjust the amount of addition to achieve the final concentration) to make a final 10 L.

### ② Preparation of cytokine 1-1 solution

A cytokine solution (C solution) was prepared by dissolving 20ug of IL-12 and 125ug of IL-18 in distilled water to make 10mL. Cytokine 1-1 solution (C1-1 solution) was prepared by dissolving 1 mL of C solution in 1000 mL of basic solution (B1 solution).
③ Solution A: A solution prepared by dissolving anti-CD16 antibody and anti-CD56 antibody in B1 solution to contain anti-CD16 antibody and anti-CD56 antibody at concentrations in a range of 0.01 to 1.5 ug/mL, respectively.
④ A1 solution: A solution prepared by dissolving anti-CD16 in C1-1 solution so that the anti-CD16 antibody is contained at a concentration in a range of 0.1 to 15 ug/mL
⑤ A2 solution: A solution prepared by dissolving anti-CD56 antibody in C1-1 solution so that the anti-CD56 antibody is contained at a concentration in a range of 0.1 to 15 ug/mL
⑥ B1 solution: A solution made with IL-2 at a concentration of 1000 to 4000 IU/mL in the suspension cell culture basic medium solution
⑦ B2 solution: A solution with twice the concentration of IL-2 than B1
⑧ C1-1 solution: A solution prepared by dissolving IL-12 and IL-18 in B1 solution to contain IL-12 at a concentration in a range of 0.5 to 5 ng/mL and IL-18 at a concentration in a range of 2 to 50 ng/mL
⑨ C5 solution: A solution with 5 times the concentration of IL-2 and IL-18 in C1-1 solution
⑩ A2 solution: A solution prepared by dissolving anti-CD3 antibody in C1-1 solution to contain anti-CD3 antibody at a concentration in a range of 0.5 to 15 ug/mL
⑪ R solution: Basic medium solution for suspension cell culture containing L-glutamine at a concentration in a range of 3 to 12 mM without cytokine or antibody

### 2. Culture process

The lymphocyte extraction and autologous plasma preparation steps were performed as in the prior patent and then cultured using the same method as described in Example 3 of the prior patent, except that some conditions were different, as described below.

Day 0: Culture was started by dissolving 1.0 × 10⁷ of PBMCs isolated from the blood into C1-1 solution. That is, the isolated PBMC was treated with 1 vial (4.5 mL) of C1-1 solution and 0.5 mL of serum, put into a T25 flask, and cultured in a CO₂ incubator. Since the C1-1 solution contains the components included in the third unit, it is the same as the third unit treated.

Day 1: After adding 1 vial (1mL) of A2 solution to the T25 flask in culture, shake the solution lightly, and putting the solution in an incubator to continue culturing. Since the A2 solution contains the components included in the third unit, it is the same as the third unit treated.

Day 2: After adding 1 vial (1 mL) of D solution to the T25 flask in culture, shake the solution lightly, and putting the solution in an incubator to continue culturing. Since the D solution contains the components included in the first unit, it is the same as the treatment of the first unit.

Day 3: After preparing 5 T25 flasks in which the cells in which the previous step was performed were cultured, 5 mL of the second unit-3 (Example 15-1) and 4.5 mL of the second unit-4 (Example 15 - 2), 4.5 mL of the second unit-5 (Example 15-3) and 4.5 mL of the second unit-6 (Example 15-4) were added. The other one was used as a control group as Comparative Example 5 without adding the second unit. After that, the culturing step was performed in the same way.

Day 4: Scrape the bottom of the cultured T25 flask with a scraper and transfer all the cells to the T75 flask. 3mL of R solution was added to the cells during cell culture, or the medium was replaced with 10mL of C1-1 solution, and after shaking gently, the culture was continued by putting it in an incubator.

Day 5: After adding 20 mL of A1 solution to the T75 flask during culture, shaking the flask gently, the flask was placed in an incubator to continue the culture.

Day 6: Scrape the bottom of the cultured T75 flask with a scraper and transfer all the cells to the T150 flask. 20mL of solution A and 3mL of autologous plasma or FBS (or similar) were added thereto, shaken gently, and placed in an incubator to continue the culture.

Day 7: After transferring all the cells in cell culture to a T150 flask, 40mL of B1 solution was added and cultured.

Day 8: After half of the 1L culture bag was picked up with forceps, the culture solution containing the cells being cultured in the T150 flask was put into this bag, and 300 mL of R solution was added.

Day 10: The forceps were removed from the 1 L CO₂ permeable bag that half had been picked up with forceps, and 300 mL of R solution was added, followed by continuous culture.

Day 11 to Day 14: 300 mL of B2 solution and 50 mL of C5 solution were added to the cells in culture. After sufficiently massaging the cells in culture to grow well, continue culturing in a 37°C, 5% CO₂ incubator.

Day 15: Immune cells were harvested by separating them into immune cells and culture medium through centrifugation (400xg). The separated culture medium is refrigerated as an immune cell culture medium.

If necessary, dissolve the isolated immune cells on Day 15 into 100 ml of B solution. After picking up half of the 1L culture bag containing the cell culture medium with forceps, add the culture medium containing the cells to this bag and add 50 mL of cytokine1-2 solution of C1-2 units to the resultant and place the resultant culture medium in a 37°C, 5% CO₂ incubator for further incubation as follows.

Day 16: After sufficiently massaging the cells in culture to grow well, continue culturing in a 37°C, 5% CO₂ incubator.

Day 17: Remove the forceps from the 1 L CO₂ permeable bag that was half been picked up with forceps, followed by continuous culture.

Day 18: After sufficiently massaging the cells in culture to grow well, continue culturing in a 37°C, 5% CO₂ incubator.

The process described above for Days 15 through 18 can be repeated one or two more times, depending on the condition of the cells, to obtain a total of 4 liters or more of culture in a single batch.

### 3. Cell number counting

To determine the effect of the immune cell proliferation kits 3 to 6 of the present disclosure, which includes at least one of the drugs for inhibiting regulatory T cells (Treg) as an active ingredient, on immune cell culture, the cell proliferation results obtained in Examples 15-1 to 15-4 and Comparative Example 4 are shown in Table 5 (unit: 1×10⁷ cells) and FIG. 5.

**[Table 5]**

| Day | 0 | 4 | 6 | 7 | 8 | 11 | 13 | 14 | 15 | 17 | 19 |
|---|---|---|---|---|---|---|---|---|---|---|---|
| Example 15-1 | 1.0 | 0.89 | 4.6 | 7.0 | 13.5 | 178 | 352 | 384 | 447 | 512 | 570 |
| Example 15-2 | 1.0 | 0.7 | 3.1 | 6.6 | 13.5 | 168 | 312 | 365 | 395 | 462 | 505 |
| Example 15-3 | 1.0 | 0.6 | 3.2 | 6.5 | 13.2 | 153 | 325 | 374 | 420 | 480 | 520 |
| Example 15-4 | 1.0 | 0.7 | 3.2 | 6.9 | 14.2 | 172 | 330 | 385 | 452 | 515 | 530 |
| Comparative Example 5 | 2.0 | 1.45 | 6.4 | 10.0 | 19.1 | 78 | 175 | 180 | 182 | 175 | 120 |

As can be seen from Table 5 and FIG. 5, in the process of culturing immune cells, Th cells were stimulated by treatment with the anti-CD3 antibody on day 2, followed by incubation with immune cell proliferation activation kit 3 containing 1 uL of dexamethasone, a steroidal injectable agent, in the medium on day 3 as in Example 15-1, and Th2, Treg, and the like. The existing medium was removed and replaced with C1 solution to remove cytokines produced by the cells, and the number of cells obtained by continued culture was compared with the number of cells obtained in Comparative Example 5, which did not use a steroidal agent, and the proliferation rate, taking into account the difference in the initial number of cells, was about 4.3 times more when cultured until day 14 and about 5.9 times more when cultured until day 17. Furthermore, as shown in Table 5, it can be seen that unlike the immune cell proliferation kit 3 containing dexamethasone, the proliferation is very good in the case of Example 15-2 to Example 15-4 when the immune cell proliferation kit 4 containing prednisolone, the immune cell proliferation kit 5 containing methylprednisolone, and the immune cell proliferation kit 6 containing betamethasone sodium phosphate are added, respectively.

### 4. Flow cytometric analysis

Flow cytometric analysis was performed on the cultured cells obtained in Example 15-1 and Comparative Example 5 according to the flow cytometry method described in experimental methods, and the results are shown in Table 6, FIG. 6A and FIG. 6B.

**[Table 6]**

| Culture method | NK cell(%) | NK cell activity (%, effector cell/K562) | |
|---|---|---|---|
| | | 30:1 | 10:1 |
| Example 15-1 | 97.08 | 91.4 | 79.6 |
| Comparative Example 5 | 92.55 | 83.5 | 72.3 |

As can be seen from Table 6, FIGS. 6A and 6B, it can be seen that Example 15-1 is higher in activity than Comparative Example 5 as well as the ratio occupied by NK cells in the proliferation culture medium. On the other hand, in Example 15-1, it can be seen that the effect may be better when the steroidal drug is added after the medium replacement on Day 4, but the effect is very good even if treated only once on Day 3. Furthermore, as already known, activated lymphocytes obtained by lymphocyte culture were used as effector cells, and a blood cancer cell line (K562) as target cells, respectively, and the ratio of activated lymphocytes to cancer cells was set to 10: 1 to measure the killing ability of the activated lymphocytes against the blood cancer cell line, the higher the proportion of NK cells in the activated lymphocytes, the higher the value, so it can be naturally predicted that the activated lymphocytes cultured by the present disclosure with a very high proportion of NK cells have a superior therapeutic effect.

### Example 16

Canine PBMCs were prepared by performing the same method as in Example 11 from blood collected from a vein of a canine, and then immune cells were cultured using the immune cell proliferation activation kit 10 according to the culture conditions in Table 7 as described below. In addition, while culturing the immune cells, the number of cells was counted using the experimental method described above, and the results are shown in Table 7 and FIG. 7.

### ① Day 0: Day of PBMC isolation from canine blood.

Lymphocytes were isolated from the blood of a healthy dog to obtain 2.24 × 10⁷ cells, which were divided into two, put into two T25 flasks, and further added 4.5 mL of the third unit-3 and 0.5 mL of plasma was to form an example flask (Example 16) and a control flask (Comparison 6), respectively, and incubated in an incubator in the conventional manner described above.

### ② Day 5

4.5 mL of the third unit-3 and 0.5 mL of plasma were further added to both the example flask and the control flask, and the second unit-1 was added only to the example flask at a concentration of 1 ug/mL in the medium, and continued culturing.

### ③ Day 6

9 mL of basic medium solution and 1 mL of plasma were added to both the example flask and the control flask and transferred to a T75 flask and continued culturing.

### ② Day 9

After adding 18 mL of basic medium solution and 2 mL of plasma to both the example flask and the control flask, transferred to a T150 flask, and continued culturing.

### ③ Day 12

Put all the culture mediums containing the cells being cultured in the example flask and the control flask into an air permeable bag containing 100mL basic medium solution, respectively, and continue culturing. At this time, the bag is used by picking up about 1/3 of the bag with forceps, and as the culture days pass, the forceps should be released appropriately.

### ③ Day 14

Move the forceps of the bag being cultured to about two-thirds of the way and continue culturing.

### ③ Day 16

Release the forceps of the bag in culture and continue culturing.

### ⑧ Day 18 to Day 21

Cells are harvested in a conventional manner.

**[Table 7]**

| Day | Add medium | Culture container | Cell count (1 x 10⁷) | |
|---|---|---|---|---|
| | | | Example 16 | Comparative Example 6 |
| 0 | Third unit-3 and plasma | T25 | 1.12 | 1.12 |
| 1 | Add first unit-1 | T25 | 1.0 | 1.0 |
| 5 | - Third unit-3 and plasma -Only in Example 16 Add second unit-1 | T25 | 0.87 | 0.86 |
| 6 | Basic medium solution 9 mL Plasma 1 mL | T75 | 1.06 | 1.06 |
| 8 | | T75 | 2.03 | 1.87 |
| 9 | Basic medium solution 18 mL Plasma 2 mL | T150 | 5.04 | 3.58 |
| 10 | | T150 | 9.68 | 6.08 |
| 12 | Basic medium solution 100 mL | bag | 27.2 | 16.5 |
| 13 | | bag | 49.2 | 30.1 |
| 14 | Basic medium solution 200 mL | bag | 88.8 | 55.6 |
| 15 | | bag | 152 | 92.1 |
| 16 | Basic medium solution 200 mL | bag | 264 | 132.2 |
| 17 | | bag | 300.8 | 152.1 |
| 20 | | bag | 324.08 | 168.9 |

As shown in Table 7 and FIG. 7, the culture results show that Example 16, in which the immune cells were first treated with the NK cell stimulating substance IL-15 included in the third unit-3 and then stimulated with the anti-CD3 antibody included in the first unit-1, and then the steroidal agent included in the second unit-1 was added to the cell culture medium on day 5, resulted in better cell proliferation than Comparative Example 6, in which the second unit-1 was not treated. In particular, it can be seen that the proliferation is significantly increased from the 8th day of culture and multiplied at least twice. Although not specifically shown, it was also confirmed that the cultures worked well when other canine PBMCs (or T cells) or anti-h-CD3 (or ca-CD3) were used instead of the first unit-1 on Day 1.

### Experimental Example 1

The results were obtained by performing the same method as Example 15-1, except that cells with a ratio of about 50% NK cells were used, and the concentration of the steroidal agent included in the immune cell proliferation activation kit was increased by about two times and added on day 3, day 4, and day 8. The results obtained by performing the same method as Comparative Example 5, except that cells with a ratio of about 50% NK cells were used, are compared, and the results are shown in FIGS. 8A and 8B, respectively.

FIGS. 8A and 8B show that the proportion of NK cells was 81.35% when the immune cell proliferation activation kit of the present disclosure was used, but 51.76% when the kit of the present disclosure was not used, so it can be seen that even if the culture was started with a high proportion of NK cells from the beginning, the proliferation rate of NK cells in the culture was further increased when the immune cell proliferation activation kit of the present disclosure was treated.

### Experimental Example 2

To test the effect of the immune cell proliferation activation kit of the present disclosure on the reactivation of cells with decreased vitality, old cells that had been cultured for 25 days and had decreased in vitality while inhibiting Treg cells according to the method of Example 15-1 were used for reactivation as follows. The supernatant was removed by centrifugation to remove the old medium and placed in a regular medium (RPMI) for culturing for about 2 hours, after which the medium was replaced with C1 solution and cultured for 3 days.

Then, the activity of the reactivated NK cells was measured, and the results are shown in Table 8.

As shown in Table 8 below, an uninhibited increase in activity was observed by Tregs, and no dramatic decrease in cell number was observed. However, a slight decrease in the number of cells, which can be thought of as a measurement error, was observed to the extent that cells naturally decrease due to aging. This method can activate NK cells in a very short period of time (1 to 3 days) and not only activates old cells but also in PBMCs immediately isolated from peripheral blood, as well as for reactivation of NK cells that have been frozen and thawed and have lost their vitality.

**[Table 8]**

| | Aged immune cells cultured for 25 days | | After 3 days of culture by treatment with C1 solution | |
|---|---|---|---|---|
| Cell | NK cells (%) | Titer (10:1, K562) | NK cells (%) | Titer(10:1, K562) |
| 1 | 57.74 | 7.3 | 69.57 | 74.51 |
| 2 | 55.52 | 41.67 | 58.06 | 92.22 |
| 3 | 55.89 | 54.13 | 57.64 | 91.75 |
| 4 | 61.41 | 66.03 | 63.44 | 92.86 |

Experimental Example 3, NK cells were cultured in the same manner as in Example 15-1, except that PBMCs were obtained from a person who had a high concentration of IL-10 in his blood, indicating that Treg cells were likely to be highly activated. The cells were then taken out on day 12, the existing culture medium was removed, and the cells were cultured for 2 days using C1 solution, after which the NK cells were isolated, and the remaining culture medium composition was obtained.

The components of the obtained culture medium composition were confirmed, and the results are shown in Table 9.

**[Table 9]**

| Cytokines | IFN-γ (ng/mL) | IL-8 (ng/mL) | IL-10 (pg/mL) | Remarks |
|---|---|---|---|---|
| A | 150.24 | 0.29 | 912.21 | Activated with C1 solution |
| B | 3.75 | 0.32 | 290.57 | Not activated by C1 solution |

Table 9 shows that when cultured for 2 days after stimulation with C1 solution on day 12 of culture, the number of cells increased well, and IFN-γ and IL-10 increased significantly, as well as only a very small amount of IL-8 was produced. On the other hand, it can be seen that the total cytokine increases less when it is separated without activating with the C1 solution. As such, even if Treg cells are human immune cells that have developed, it can be seen that the number of cells does not decrease but increases, and a large amount of IFN-γ and IL-10 is produced when stimulated with C1 solution on the 12th day of culture after treatment with the immune cell proliferation activation kit of the present disclosure.

On the other hand, IL-8 is a cytokine that recruits neutrophils and is often associated with inflammation, and IL-10 is known to induce macrophages into M2b type and play a role in removing inflammation, so the culture medium composition obtained is effective in improving skin problems as well as treating skin diseases caused by inflammation.

Although the present disclosure is described with reference to the preferred embodiment as described above, the present disclosure is not limited thereto, and various changes and modifications may be made by those skilled in the art without departing from the spirit of the present disclosure.

## Claims

1. An immune cell proliferation activation kit comprising:
a first unit comprising a T cell stimulating substance; and
a second unit comprising a steroidal agent.

2. The immune cell proliferation activation kit of claim 1, wherein the first unit is formed such that the T cell stimulating substance is contained in a basic medium solution at a concentration of 0.1 to 12 ug/mL when the T cell stimulating substance is an antibody, and T cell stimulating substance is contained at a concentration of at least 1×10⁵ cells/mL when the T cell stimulating substance is a feeder cell, wherein the antibody is one or more selected from the group consisting of an anti-CD3 antibody, an anti-CD4 antibody, and an anti-CD28 antibody.

3. The immune cell proliferation activation kit of claim 1, wherein the second unit is formed such that the steroidal agent is contained in a basic medium solution at a concentration of 0.02 ug/ml or more.

4. The immune cell proliferation activation kit of claim 3, wherein the steroidal agent is glucocorticoids, and the steroidal agent is at least one selected from the group consisting of cortisol, cortisone, prednisolone, prednisone, methylprednisolone, dexamethasone, betamethasone, triamcinolone, fludrocortisone acetate, and deoxycorticosterone acetate.

5. The immune cell proliferation activation kit of claim 1, wherein the steroidal agent acts to inhibit the activity of regulatory T cells (Treg) to cause NK cells to proliferate.

6. The immune cell proliferation activation kit of claim 1, further comprising:
a third unit comprising a NK cell stimulating substance, wherein the third unit is formed such that the NK cell stimulating substance is contained in a basic medium solution at a concentration of 0.2 ng/mL to 10 µg/mL.

7. The immune cell proliferation activation kit of claim 6, wherein the NK cell stimulating substance is at least one selected from the group consisting of an anti-CD16 antibody, anti-CD56 antibody, IL-12, IL-15, and IL-18.

8. An immune cell culture method comprising:
a first culturing step in which peripheral blood mononuclear cells (PBMC) isolated from blood are suspended in a basic medium solution and then cultured for 18 to 72 hours while being treated with a first unit comprising a T cell stimulating substance;
a second culturing step in which a first culture solution having undergone the first culturing step is treated with a second unit comprising a steroidal agent and is then cultured for 18 to 25 hours; and
a third culturing step in which a second culture solution having undergone the second culturing step is cultured while either one or both of a basic medium solution and a fetal bovine serum (FSB) are added to the second culture solution at intervals of 24 hours or more starting from a certain point, and the second culture solution is transferred to a larger culture container as the number of the cells increases.

9. The method of claim 8, wherein in the first culturing step, the cells are treated with a third unit comprising an NK cell stimulating substance preliminarily or simultaneously with the treatment with the first unit.

10. The method of claim 9, wherein the third unit is formed such that the NK cell stimulating substance is contained at a concentration of 0.2 ng/mL to 10 ug/mL in a basic medium solution, and
the NK cell stimulating substance is at least one selected from the group consisting of anti-CD16 antibodies, anti-CD56 antibodies, IL-12, IL-15, and IL-18.

11. The method of claim 8, wherein the first unit is formed such that the T cell stimulating substance is contained in a basic medium solution at a concentration of 0.1 to 12 ug/mL when T cell stimulating substance is an antibody, and the T cell stimulating substance is contained in a concentration of 1×10⁵ cells/mL or more when the T cell stimulating substance is a feeder cell, wherein the antibody is at least one selected from the group consisting of an anti-CD3 antibody, an anti-CD4 antibody, and an anti-CD28 antibody, and
the second unit is formed such that steroidal agent is contained at a concentration of 0.02 ug/mL or more in a basic medium solution, wherein the steroidal agent is glucocorticoid, and is at least one selected from the group consisting of cortisol, cortisone, prednisolone, prednisone, methylprednisolone, dexamethasone, betamethasone, triamcinolone, fludrocortisone acetate, and deoxycorticosterone acetate.

12. The method of claim 11, wherein the first unit is used for the treatment of the PBMC suspended in the basic medium solution on the 0th day of culture, the 1st day of culture, or the 2nd day of culture, and the second unit is used for treatment 20 to 25 hours after the first culture solution is treated with the first unit.

13. The method of claim 12, wherein the second culture solution having undergone the second culturing step is treated with the second unit one more time after 5 days of culture.

14. A method of culturing pet immune cell, the method comprising:
a first culturing step in which peripheral blood mononuclear cells (PBMC) isolated from canine blood are suspended in a basic medium solution and is then cultured for 4 days while being treated with a third unit comprising NK cell stimulating substance and a first unit comprising T cell stimulating substance sequentially or simultaneously;
a second culturing step in which a first culture solution having undergone the first culturing step is treated with a second unit comprising a steroidal agent and a third unit sequentially or simultaneously and is then cultured for 18 to 25 hours; and
a third culturing step in which a second culture solution having undergone the second culturing step is cultured while either one or both of a basic medium solution and a fetal bovine serum (FSB) are added to the second culture solution at intervals of 24 hours or more starting from a certain point, and the second culture solution is transferred to a larger culture container as the number of the cells increases.

15. The method of claim 14, wherein the first unit is formed such that the T cell stimulating substance is contained in a basic medium solution at a concentration of 0.1 to 12 ug/mL when the T cell stimulating substance is an antibody, and the T cell stimulating substance is contained at a concentration of at least 1×10⁵ cells/mL when the T cell stimulating substance is a feeder cell, wherein the antibody is one or more selected from the group consisting of an anti-CD3 antibody, an anti-CD4 antibody, and an anti-CD28 antibody,
the second unit is formed such that steroidal agent is contained at a concentration of 0.02 µg/mL or more in a basic medium solution, wherein the steroidal agent is glucocorticoid, and is at least one selected from the group consisting of cortisol, cortisone, prednisolone, prednisone, methylprednisolone, dexamethasone, betamethasone, triamcinolone, fludrocortisone acetate, and deoxycorticosterone, and
the third unit is formed such that the NK cell stimulating substance in a basic medium solution at a concentration of 0.2 ng/mL to 10 ug/mL, and the NK cell stimulating substance is at least one selected from the group consisting of an anti-CD16 antibody, anti-CD56 antibody, IL-12, IL-15, and IL-18.
